# EUROPEAN PATENT SPECIFICATION

(11) **EP 4 419 182 B1**
(45) Date of publication and mention of the grant of the patent: **02.12.2026**
(21) Application number: 22883113.7
(22) Date of filing: 19.10.2022
(51) Int. Cl.: A61M 25/00, A61B 17/00, A61B 17/12

(54) **EMBOLIZATION MICROCATHETER FOR DELIVERY OF BEADS TO PERIPHERAL VESSELS**
EMBOLISATIONSMIKROKATHETER ZUR ABGABE VON KÜGELCHEN AN PERIPHERE GEFÄSSE
MICROCATHÉTER D'EMBOLISATION POUR L'ADMINISTRATION DE BILLES À DES VAISSEAUX PÉRIPHÉRIQUES

(30) Priority: 20.10.2021 IL 28742021; 12.05.2022 US 202263341014 P
(43) Date of publication of application: 28.08.2024
(73) Proprietor: Accurate Medical Therapeutics Ltd., 7608802 Rehovot (IL)
(72) Inventor: CASHDAN, Judy, 7312700 Hashmonaim (IL); DAGAN, Tom, 8496500 Omer (IL); ZIPORY, Yuval, 7175472 Modiin (IL); HARBATER, Osnat, 4358142 Raanana (IL); MILLER, Eran, 7680300 Moshav Beit Elazari (IL)
(74) Representative: AWA Denmark A/S
(86) International application number: PCT/IL2022/051107
(87) International publication number: WO 2023/067601

(56) References cited:
- EP-B1- 2 766 084
- WO-A1-2021/035042
- IL-B- 276 416
- IL-B- 276 416
- US-A1- 2013 123 752
- US-A1- 2020 086 082
- US-B2- 8 096 985

## Description

### TECHNICAL FIELD

The present disclosure generally relates to the field of microcatheters for embolization, specifically to embolization catheters suitable for navigating within blood vessels for delivery of embolization beads to peripheral blood vessels.

### BACKGROUND

Transarterial embolization therapy, tumor embolization, or transcatheter arterial embolization (TAE), involves administration of embolization material (which may include chemotherapeutics or/and radiotherapeutics) directly to a tumor (for example, liver tumors), via a microcatheter.

Embolization of tumors is typically performed utilizing microcatheters due to the requirement for selectively affecting the tumor while preventing, as much as possible, damage to healthy tissue. A major problem associated with embolization is "non-target embolization", where the embolic material travels to blood vessels, other than those directly feeding the target tumor or tumor region, thus damaging healthy tissues, resulting in unpleasant and even hazardous outcomes.

During embolization, the embolization catheter must be advanced and navigate through small and often tortuous vessels. Accessibility to these vessels is difficult, if not precluded, using standard microcatheters. Moreover, tracking the direction of the microcatheter's progress within the blood vessels is limited due to the structure of the microcatheters.

US 2020/086082 A1 discloses a guide catheter with a deformation zone to ease navigation through blood vessels. The guide catheter comprises a tubular catheter body with one or more conduits and a pull lumen containing a pull member. The pull member is attached distally to catheter body such that, when pulled proximally, pull member will cause catheter body to bend. When the deformation zone is straightened, triangular sections of a first layer segment of the pull member are stretched or relieved of compression as an angle is increased by the angular separation of triangular sections of a second layer segment of the pull member. When the deformation zone is acutely bent, triangular sections of the first layer segment of the pull member are compressed as the angle is decreased by the angular reduction of triangular sections of the second layer segment of the pull member.

There thus remains a need for microcatheters capable of delivering embolization beads to small and tortuous blood vessels and facilitate navigation and reliable real time tracking during the embolization procedure.

### SUMMARY OF THE INVENTION

The present disclosure relates to an embolization microcatheter according to claim 1 which is suitable for navigation in small and tortuous blood vessels, while also facilitating reliable real time tracking during the embolization procedure, as well as to a method for producing such an embolization microcatheter according to claim 15. Advantageous embodiments are described in the dependent claims.

This is advantageously achieved by the unique structure of the embolization microcatheters, which has an elongated main body and a distal section, which includes an off-setting portion and a winding portion. The winding portion has about half a loop of a three-dimensional spiral, such that a longitudinal axis of the winding portion is offset and/or disposed at an acute angle relative to a longitudinal axis of the elongated main body.

Advantageously, the winding portion at the most distal of the embolization microcatheter, allows a "screw like" motion and thus facilitates advancement of the microcatheter within the blood vessel even in tortuous sections.

Moreover, the three-dimensional structure of the embolization microcatheter, particularly the off-setting of the distal winding portion, as disclosed herein in accordance with some embodiments, advantageously provides a "line-of-sight" of the microcatheter's tip as it progresses through the blood vessel. This may not be facilitated in a standard two-dimensional microcatheter, where the tip section may be hindered by the microcatheter's elongated main body and thus not seen when observing it from a proximal point of view.

Furthermore, the three-dimensional structure of the embolization microcatheter, as disclosed herein in accordance with some embodiments, may advantageously facilitate the securing the microcatheter within the blood vessel by contacting at least two points of the blood vessel wall. Such contact points contribute to the stabilization of the microcatheter in the blood vessel, which is of great importance, particularly during the delivery of embolization beads.

In operation, when the embolization microcatheters, disclosed herein in accordance with some embodiments, is inserted to blood vessel, it progresses in a "screw like" motion facilitated by the winding portion of its distal section. While the user advances the microcatheters within the often small and tortuous blood vessel, the winding portion, which is offset from the elongated main body, can be observed during the procedure. This facilitates navigation of the microcatheter within the blood vessel. During the procedure the user can also utilize the three-dimensional structure of the embolization microcatheter to contact the blood vessel wall at two or more points. This allows stabilization of the microcatheter within the blood vessel and facilitates safer and more accurate performance of the procedure.

According to the invention, there is provided an embolization microcatheter comprising an elongated main body and a distal section, the distal section comprising an off-setting portion and a winding portion; wherein the winding portion comprises about half a loop of a three-dimensional spiral, such that a longitudinal axis of the winding portion is offset and/or disposed at an acute angle relative to a longitudinal axis of the elongated main body; wherein the off-setting portion is disposed at an angle of between about 60° and about 120° degrees relative to the longitudinal axis of the elongated main body. According to some embodiments, the winding portion is configured to maneuver the microcatheter longitudinally in a screw-like motion thereby, preventing collapsing, bending kinking, backwards movement, whipping, lashing or any combination thereof of the distal section of the microcatheter. Advantageously, the screw-like motion enables controlled and/or precise steering of the distal section into specific vessels, such as small side branches, which are typically difficult to reach without numerous back and forth movements.

According to some embodiments, the off-setting portion is curved.

According to some embodiments, the longitudinal axis of the winding portion may be offset by between about 0.5 mm and about 5 mm relative to the longitudinal axis of the elongated main body.

According to some embodiments, the acute angle, at which the longitudinal axis of the winding portion is disposed relative to the longitudinal axis of the elongated main body, may be between about 10° and about 60°.

According to some embodiments, the length of the winding portion measures between about 5 mm and about 20 mm. According to some embodiments, the ratio of the length of the winding portion to a radius or mean radius, defined by the half a loop, is between about 2 and about 20.

According to some embodiments, the three-dimensional spiral is right-handed. According to some embodiments, the three-dimensional spiral is left- handed. According to some embodiments, the three-dimensional spiral is a helix.

According to some embodiments, the distal section may be pre-shaped/ pre-formed, for example, in the winding/curled/coiled structure.

According to some embodiments, the main body and the off-setting portion together may define a non-planar curve.

According to some embodiments, the elongated main body may further include a filter section including a plurality of side openings formed in a wall of the elongated main body. The side openings may be in the form of slits having a length between about 200 µm and about 800 µm and a width between about 25 µm and about 100 µm.

According to some embodiments, the embolization microcatheter may include a wall having an inner layer, an outer layer, and a skeleton intercalated between the inner layer and the outer layer. According to some embodiments, the outer layer section of the outer layer, on the distal section, may be more flexible than an outer layer section of the outer layer, on the elongated main body.

According to some embodiments, the distal section may be dimensioned such that, when inserted into a tortuous blood vessel, different portions of the distal section contact opposite sides of the tortuous blood vessel at different longitudinal positions of the tortuous blood vessel.

According to some embodiments, the embolization microcatheter may be configured for guide-wire free navigation.

According to some embodiments, the off-set portion is configured to minimize interference of a user's field of view by the winded portion.

According to some embodiments, there is provided a method for performing an embolization procedure, the method includes: inserting to a blood vessel and advancing a embolization microcatheter and performing an embolization procedure wherein the embolization microcatheter includes an elongated main body and a distal section, the distal section comprising an off-setting portion and a winding portion; wherein the winding portion comprises about half a loop of a three-dimensional spiral, such that a longitudinal axis of the winding portion is offset and/or disposed at an acute angle relative to a longitudinal axis of the elongated main body; and wherein the off-setting portion is disposed at an angle of between about 60° and about 120° degrees relative to the longitudinal axis of the elongated main body.

According to some embodiments, there is provided a method for producing an embolization microcatheter distal section that defines a non-planar curve, the method comprising: providing a 3-dimensional mandrel comprising an off-setting portion and a winding portion, wherein the winding portion comprises about half a loop of a three-dimensional spiral, such that a longitudinal axis of the winding portion is offset and/or disposed at an acute angle relative to a longitudinal axis of the elongated main body; and wherein the off-setting portion is disposed at an angle of between about 60° and about 120° degrees relative to the longitudinal axis of the elongated main body; in a heat chamber forming the embolization microcatheter tube on the mandrel, thereby producing the embolization microcatheter having the shape of the mandrel.

Certain embodiments of the present disclosure may include some, all, or none of the above characteristics. One or more technical advantages may be readily apparent to those skilled in the art from the figures, descriptions and claims included herein. Moreover, while specific characteristics have been enumerated above, various embodiments may include all, some or none of the enumerated characteristics.

In addition to the exemplary aspects and embodiments described above, further aspects and embodiments will be further expanded upon in the figures and the following detailed descriptions.

### BRIEF DESCRIPTION OF THE FIGURES

The features, nature and advantages of the present disclosure will become more apparent from the detailed description set forth below when taken in conjunction with the drawings in which like reference characters identify correspondingly throughout. Identical structures elements or parts that appear in more than one figure are generally labeled with the same number in all the figures in which they appear. Alternatively, elements or parts that appear in more than one figure may be labeled with different numbers in the different figures in which they appear. The dimensions of the components and features in the figures were chosen for convenience and clarity of presentation and are not necessarily shown to scale. The figures are listed below.
**Figure 1** presents a schematic, perspective view of an embolization microcatheter including an elongated main body and a distal section, according to some embodiments;
**Figure 2A** presents a schematic, side-view of the distal section of the embolization microcatheter of **Figure 1** from a point-of-view directed along the negative z-axis (indicated), according to some embodiments;
**Figure 2B** presents a schematic, side-view of the distal section of the embolization microcatheter of **Figure 1** from a point-of-view directed along the positive z-axis, according to some embodiments;
**Figure 3A** presents a schematic, side-view of the distal section of the embolization microcatheter of **Figure 1** from a point-of-view directed along the negative *y*-axis (indicated), according to some embodiments;
**Figure 3B** presents a schematic, side-view of the distal section of the embolization microcatheter **of** **Figure 1** from a point-of-view directed along the positive *y*-axis, according to some embodiments;
**Figure 4A** presents a schematic, front-view of the embolization microcatheter of **Figure 1****,** according to some embodiments;
**Figure 4B** presents a schematic, back-view of the embolization microcatheter of **Figure 1****,** according to some embodiments;
**Figure 5A** presents a schematic, side-view of the embolization microcatheter of **Figure** 1 from a point-of-view pointing substantially midway along a direction between the negative *y*-axis and the negative z-axis on the yz-plane, according to some embodiments;
**Figure 5B** presents a schematic, side-view of the embolization microcatheter of **Figure 1** from a point-of-view pointing substantially midway along a direction between the positive *y*-axis and the positive z-axis on the yz-plane, according to some embodiments;
**Figure 6A** presents a schematic, cross-sectional view of the embolization microcatheter of **Figure 1** from the point-of-view of **Figure 5A****,** the cross-section bisects the main body along a plane perpendicular to the direction defined by the point-of-view, according to some embodiments;
**Figure 6B** presents a schematic, cross-sectional view of the embolization microcatheter of **Figure 1** from the point-of-view of **Figure 5B****,** the cross-section bisects the main body along a plane perpendicular to the direction defined by the point-of-view, according to some embodiments;
**Figure 7** presents a histogram of the average peak tracking force of the embolization microcatheter disclosed herein according to some embodiments (designated by the name of "Drakon"), in comparison to other embolization microcatheter;
**Figure 8** presents a histogram of the advancing distance through tortuous anatomy achieved by the embolization microcatheter disclosed herein according to some embodiments (designated by the name of "Drakon"), in comparison to other embolization microcatheter; and
**Figure 9** presents a histogram of the tip response to torque of the embolization microcatheter disclosed herein according to some embodiments (designated by the name of "Drakon"), in comparison to other embolization microcatheter.

### DETAILED DESCRIPTION OF THE INVENTION

The detailed description set forth below, in connection with the appended drawings, is intended as a description of various configurations and is not intended to represent the only configurations in which the concepts described herein may be practiced. The detailed description includes specific details for the purpose of providing a thorough understanding of the various concepts. However, it will also be apparent to one skilled in the art that these concepts may be practiced without specific details being presented herein. In some instances, well-known features may be omitted or simplified in order to avoid obscuring the disclosure.

One of the main challenges of embolization microcatheters is navigation in small and tortuous blood vessels, while also facilitating reliable real time tracking of the microcatheter's advancement during the embolization procedure.

Advantageously, these requirements are met by the herein disclosed microcatheter and its structural characteristics.

There is thus provided herein, in accordance with some embodiments, an embolization microcatheter including an elongated main body and a distal section, the distal section comprising an off-setting portion and a winding portion, wherein the winding portion comprises about half a loop of a three-dimensional spiral, such that a longitudinal axis of the winding portion is offset and/or disposed at an acute angle relative to a longitudinal axis of the elongated main body, and wherein the off-setting portion is disposed at an angle of between about 60° and about 120° degrees (for example, between about 60° and about 80°, between about 80° and about 100° or between about 100° and about 120°, each possibility presets a separate embodiment) relative to the longitudinal axis of the elongated main body. The off-setting portion may be curved.

According to some embodiments, the longitudinal axis of the winding portion may be offset by between about 0.5 mm and about 5 mm (for example, between about 0.5 mm and about 1 mm, between about 1 mm and about 3 mm or between about 3 mm and about 5 mm, each possibility presets a separate embodiment) relative to the longitudinal axis of the elongated main body.

According to some embodiments, the acute angle, at which the longitudinal axis of the winding portion is disposed relative to the longitudinal axis of the elongated main body, is between about 10° and about 60°, for example, between about 10° and about 30°, between about 30° and about 40° or between about 40° and about 60°, each possibility presets a separate embodiment.

According to some embodiments, the length of the winding portion measures between about 5 mm and about 20 mm, for example, between about 5 mm and about 10 mm, between about 10 mm and about 15 mm or between about 15 mm and about 20 mm, each possibility presets a separate embodiment.

According to some embodiments, the ratio of the length of the winding portion to a radius or mean radius, defined by the half a loop, is between about 2 and about 20, for example, between about 2 and about 5, between about 5 and about 10 or between about 10 and about 20, each possibility presets a separate embodiment.

According to some embodiments, the microcatheter 100 may have a length of at least 50 cm, at least 60 cm, at least 75 cm, or at least 1m. Each possibility is a separate embodiment. Each possibility is a separate embodiment.

According to some embodiments, the three-dimensional spiral may be right-handed. According to some embodiments, the three-dimensional spiral may be left-handed. According to some embodiments, the three-dimensional spiral may be a helix. According to some embodiments, the distal section may be pre-shaped.

According to some embodiments, the main body and the off-setting portion together define a non-planar curve.

According to some embodiments, the elongated main body may further include a filter section, which includes a plurality of side openings formed in a wall of the elongated main body. The side openings may be in the form of slits having a length between about 200 µm and about 800 µm and a width between about 25 µm and about 100 µm, for example, having a length between about 200 µm and about 300 µm and a width between about 75 µm and about 100 µm, or having a length between about 600 µm and about 800 µm and a width between about 25 µm and about 50 µm or having a length between about 500 µm and about 800 µm and a width between about 50 µm and about 100 µm or having a length between about 200 µm and about 400 µm and a width between about 25 µm and about 50 µm, each possibility presets a separate embodiment.

According to some embodiments, the embolization microcatheter has a wall, which includes an inner layer, an outer layer, and a skeleton intercalated between the inner layer and the outer layer. An outer layer section of the outer layer, on the distal section, may be more flexible than an outer layer section of the outer layer, on the elongated main body.

According to some embodiments, the distal section may be dimensioned such that, when inserted into a tortuous blood vessel, different portions of the distal section contact opposite sides of the tortuous blood vessel at different longitudinal positions of the tortuous blood vessel.

As used herein, the terms "embolization", "transcatheter embolization", "transcatheter arterial embolization" and "TAE" may be used interchangeably and refer to the passage and lodging of an embolus within the bloodstream for therapeutic purposes, for example, as a hemostatic treatment of bleeding or as a treatment for some types of cancer by deliberately blocking blood vessels to starve the tumor cells.

As used herein, in accordance with some embodiments, the term "winding portion" may refer to a section of the microcatheter having a non-straight, curved, twisted, bent, spiral, coiled and/or partially coiled shape and/or having one or more loops/partial loops.

As used herein, in accordance with some embodiments, the term "off-setting portion" may refer to a section of the microcatheter connecting between the elongated main body and the winding portion of the microcatheter and shaped to create an offset and/or an (acute) angle between the longitudinal axis of the winding portion and the longitudinal axis of the elongated main body.

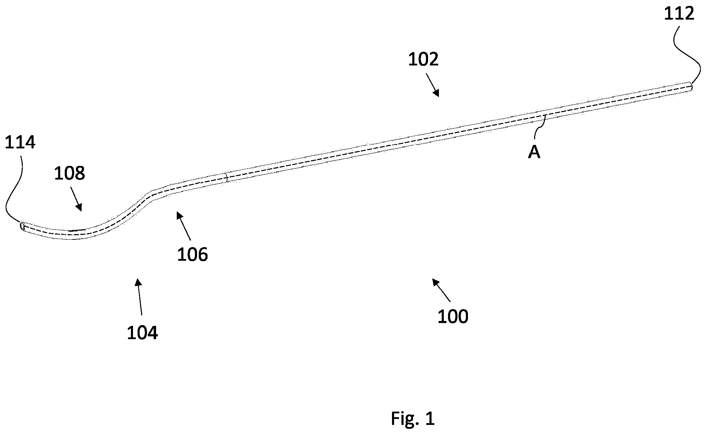

Reference is now made to **Figure 1****,** which presents a schematic, perspective view of an embolization microcatheter **100** including an elongated main body **102** and a distal section **104,** according to some embodiments. Distal section **104** includes an off-setting portion **106** and a winding portion **108.** Embolization microcatheter **100** extends from a proximal end opening **112** to a distal end opening **114.**

As used herein, the terms "distal end opening" and "proximal end opening" refer to the end openings of the microcatheter leading into the lumen thereof.

Dashed line "A" represents a central longitudinal axis of embolization microcatheter **100** extending from elongated main body **102** and distal section **104,** which includes off-setting portion **106** and winding portion **108.**

Off-setting portion **106** is disposed at an angle (for example, of between about 60° and about 120° degrees) relative to the longitudinal axis of elongated main body **102** and winding portion **108** forms about half a loop of a three-dimensional spiral, such that the longitudinal axis of winding portion **108** is offset relative to the longitudinal axis of elongated main body **102.**

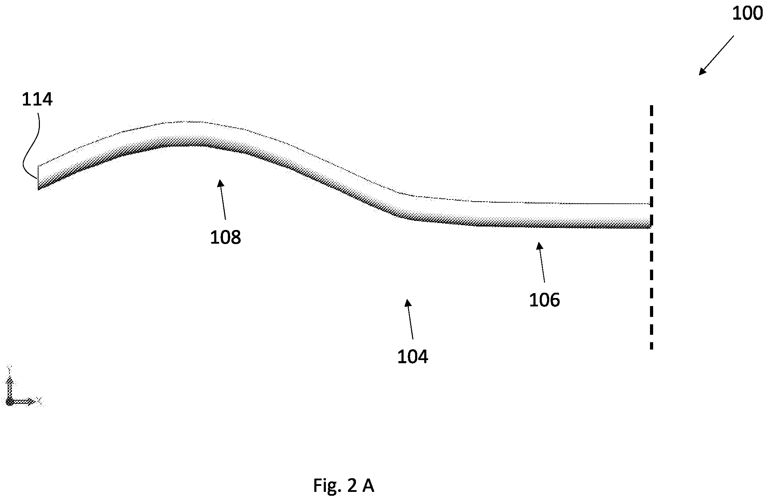

Reference is now made to **Figures 2A-6B****,** which present various views of embolization microcatheter **100,** therefore the elements or parts numbers of **Figure 1** apply to the same elements or parts of **Figures 2A-6B****.**

**Figure 2A** presents a schematic, side-view of the distal section of embolization microcatheter **100** of **Figure 1** from a point-of-view directed along the negative z-axis (indicated), according to some embodiments. It is noted that off-setting portion **106** (and elongated main body **102)** continues beyond the dashed vertical line but is not shown in the figure.

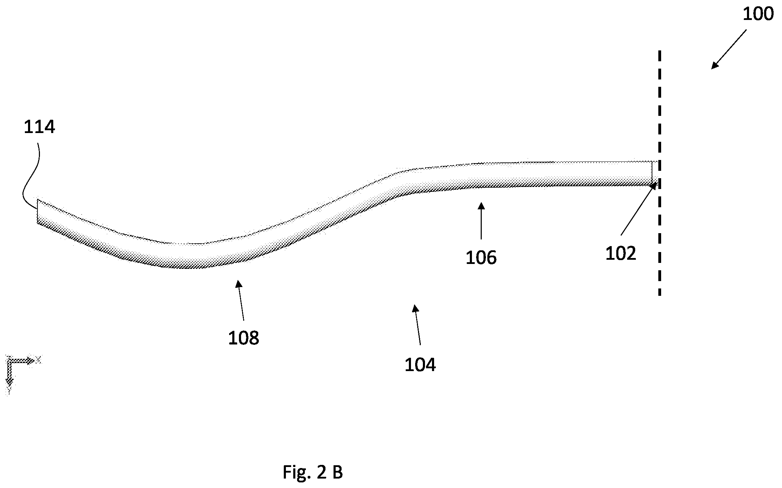

**Figure 2B** presents a schematic, side-view of the distal section of embolization microcatheter **100** of **Figure 1** from a point-of-view directed along the positive z-axis (indicated), according to some embodiments. It is noted that elongated main body **102** continues beyond the dashed vertical line but is not shown in the figure.

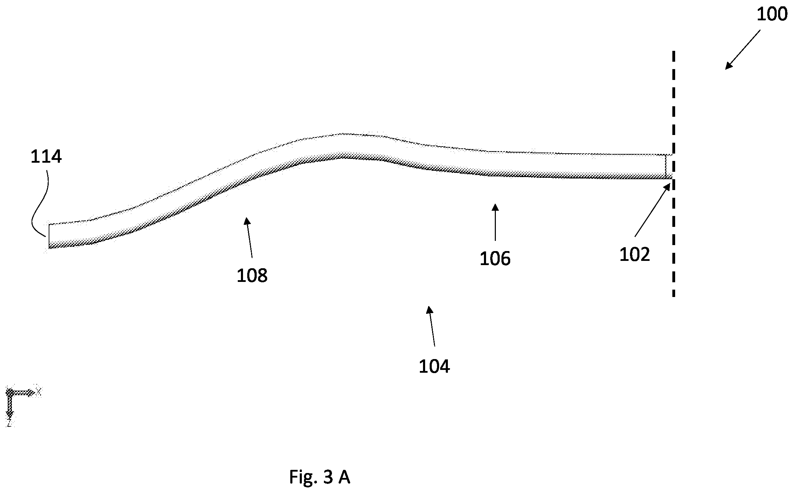

**Figure 3A** presents a schematic, side-view of the distal section of embolization microcatheter **100** of **Figure 1** from a point-of-view directed along the negative *y*-axis (indicated), according to some embodiments. It is noted that elongated main body **102** continues beyond the dashed vertical line but is not shown in the figure.

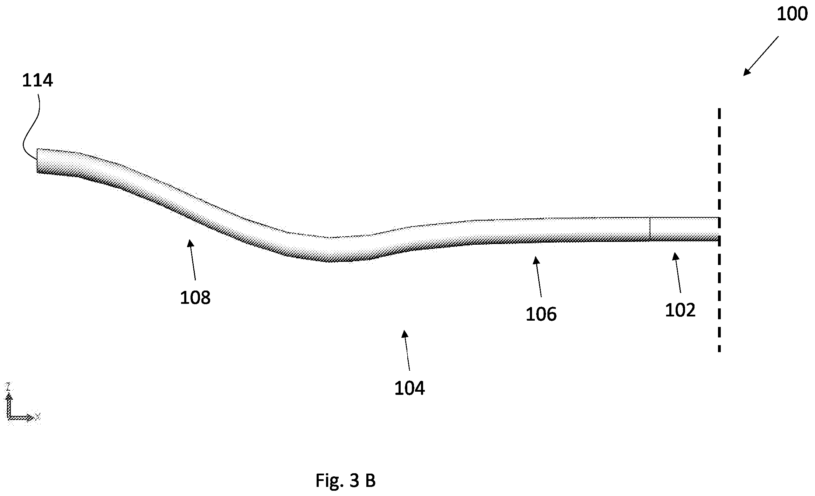

**Figure 3B** presents a schematic, side-view of the distal section of embolization microcatheter **100** of **Figure 1** from a point-of-view directed along the positive *y*-axis (indicated), according to some embodiments, according to some embodiments. It is noted that elongated main body **102** continues beyond the dashed vertical line but is not shown in the figure.

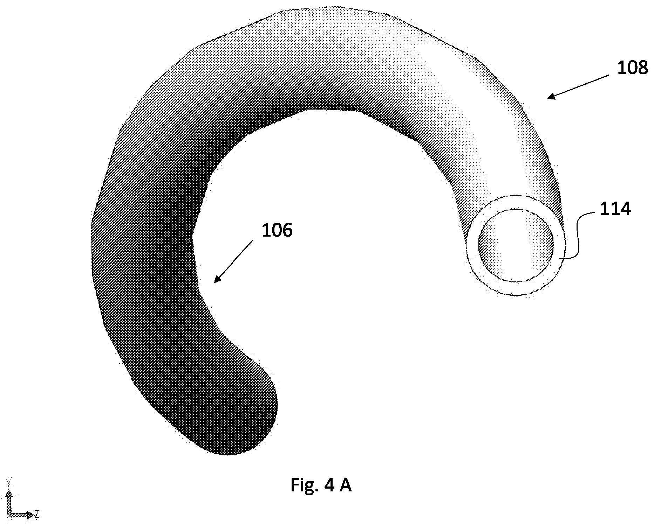

**Figure 4A** presents a schematic, front-view (looking towards distal end opening **114)** of the embolization microcatheter **100** of **Figure 1****,** according to some embodiments.

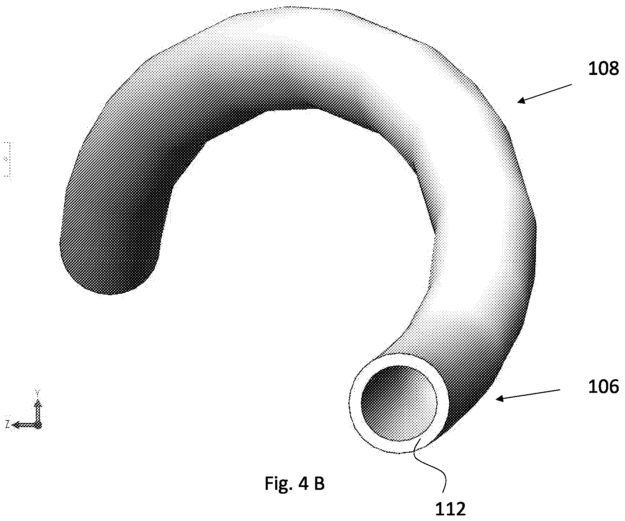

**Figure 4B** presents a schematic, back-view (looking towards proximal end opening **112)** of the embolization microcatheter **100** of **Figure 1****,** according to some embodiments. It is noted that in this point of view, elongated main body **102** cannot be seen as it is "hidden" behind proximal end opening **112,** while only off-setting portion **106** and winding portion **108** are visible.

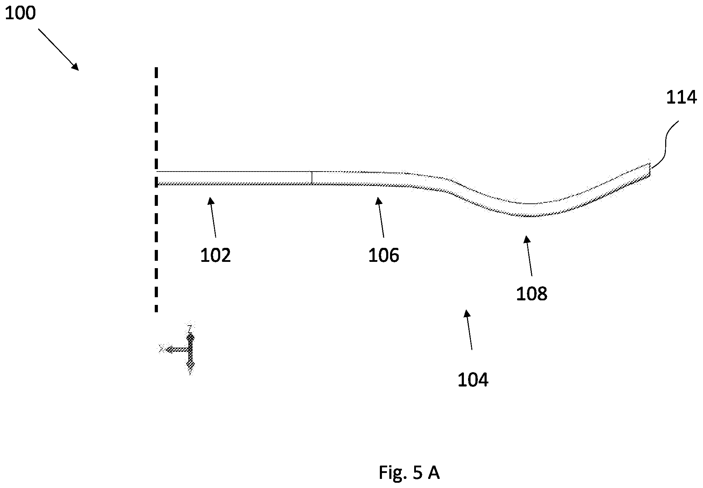

**Figure 5A** presents a schematic, side-view of embolization microcatheter **100** of **Figure 1** from a point-of-view pointing substantially midway along a direction between the negative*y*-axis and the negative z-axis on the yz-plane, according to some embodiments. It is noted that elongated main body **102** continues beyond the dashed vertical line but is not shown in the figure.

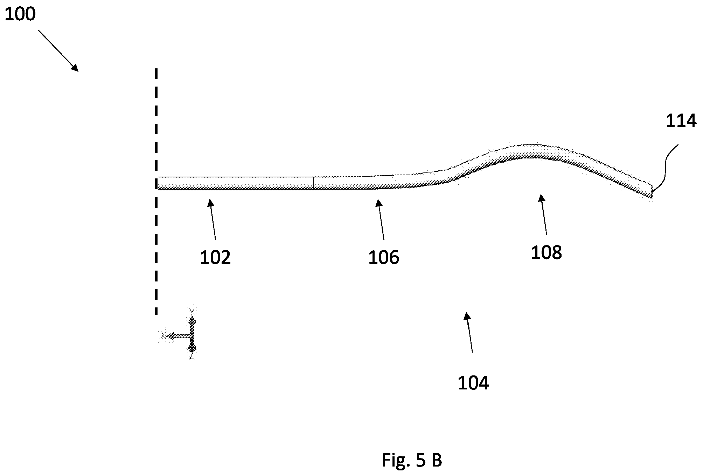

**Figure 5B** presents a schematic, side-view of the embolization microcatheter of **Figure 1** from a point-of-view pointing substantially midway along a direction between the positive *y*-axis and the positive z-axis on the yz-plane, according to some embodiments. It is noted that elongated main body **102** continues beyond the dashed vertical line but is not shown in the figure.

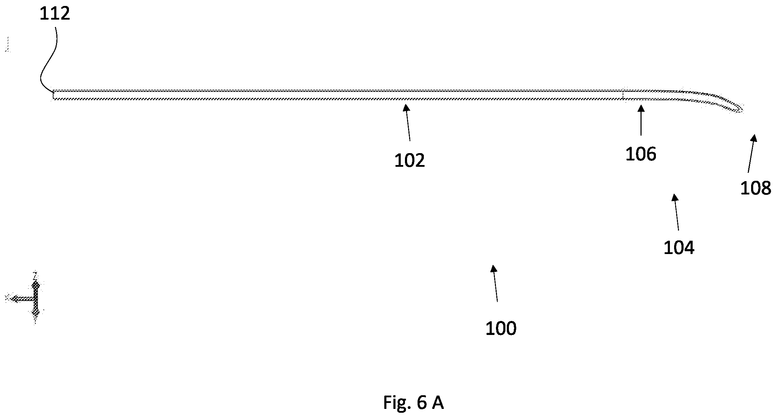

**Figure 6A** presents a schematic, cross-sectional view of embolization microcatheter **100** of **Figure 1** from the point-of-view of **Figure 5A****,** the cross-section bisects the main body along a plane perpendicular to the direction defined by the point-of-view, according to some embodiments. It is noted that in this cross-sectional point of view, winding portion **108** (or at least a portion thereof) is not visible due to its angle.

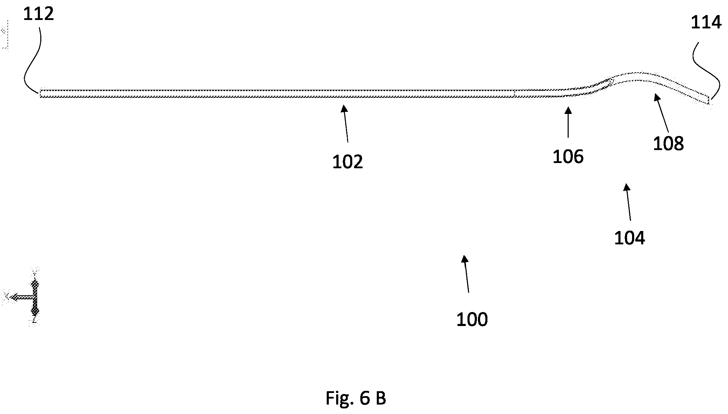

**Figure 6B** presents a schematic, cross-sectional view of embolization microcatheter **100** of **Figure 1** from the point-of-view of **Figure 5B****,** the cross-section bisects the main body along a plane perpendicular to the direction defined by the point-of-view, according to some embodiments. In this cross-sectional point of view, as opposed to the cross-sectional point of view of **Figure 6A****,** winding portion **108** is not visible, however, not cross- sectioned.

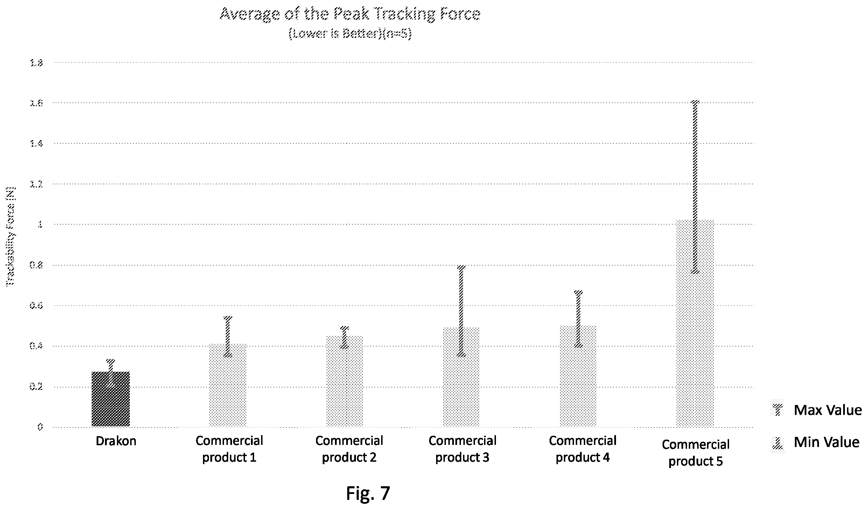

Reference is now made to **Figures 7-11,** which present experimental results demonstrating the better performance of the embolization microcatheter disclosed herein according to some embodiments (designated by the name of "Drakon") in comparison to other embolization microcatheters.

**Figure 7** presents a histogram of the average peak tracking force of the embolization microcatheter disclosed herein according to some embodiments (designated by the name of "Drakon"), in comparison to other embolization microcatheters (Test Protocol RD-039; n=5). The lowest trackability force [N] is obtained for the Drakon compared to other commercial embolization microcatheters, designated as commercial products 1-5. The lower trackability force is indicative of better maneuverability.

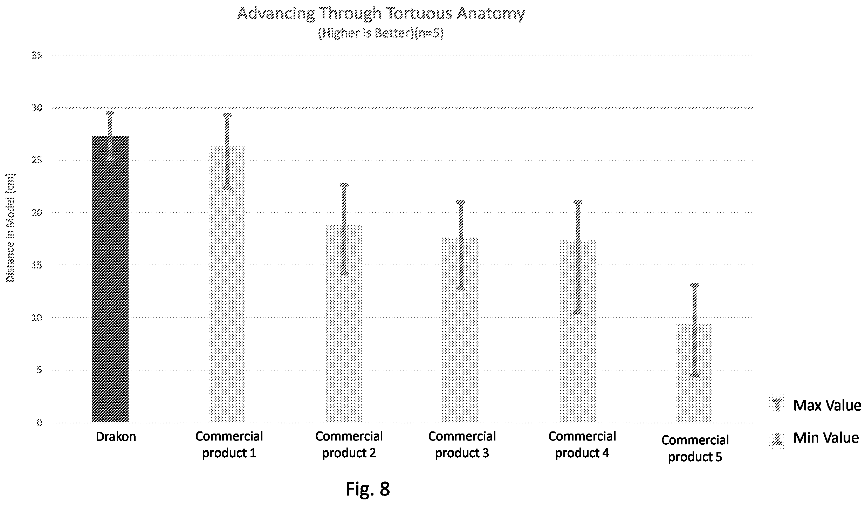

**Figure 8** presents a histogram of the advancing distance through tortuous anatomy achieved by the embolization microcatheter disclosed herein according to some embodiments (designated by the name of "Drakon"), in comparison to other commercial embolization microcatheters (Test Protocol RD-055; n=5). It can be seen that the Drakon embolization microcatheter is capable of advancing in tortuous anatomy (model) farther than the other microcatheters tested.

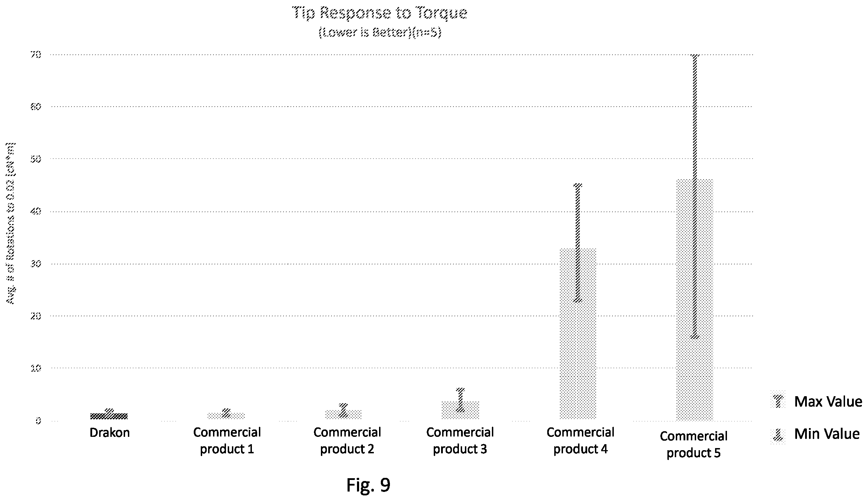

**Figure 9** presents a histogram of the tip response to torque of the embolization microcatheter disclosed herein according to some embodiments (designated by the name of "Drakon"), in comparison to other commercial embolization microcatheters (Test Protocol RD-018; n=5). As can be seed from the histogram, the Dragon shows the lowest tip response to torque, measures by the average number of rotations to 0.02cN*m.

As used herein, the terms "approximately" and "about" refer to +/-10%, or +/-5%, or +-2% vis-à-vis the range to which it refers. Each possibility is a separate embodiment.

While a number of exemplifying aspects and embodiments have been discussed above, those of skill in the art will envisage certain modifications, additions and subcombinations thereof.

## Claims

1. An embolization microcatheter (100) comprising an elongated main body (102) and a distal section (104), the distal section (104) comprising an off-setting portion (106) and a winding portion (108);
wherein the winding portion (108) comprises about half a loop of a three-dimensional spiral, such that a longitudinal axis of the winding portion (108) is offset and/or disposed at an acute angle relative to a longitudinal axis of the elongated main body (102);
wherein the off-setting portion (106) is disposed at an angle of between about 60° and about 120° degrees relative to the longitudinal axis of the elongated main body (102), and
wherein the winding portion (108) is configured to maneuver the microcatheter (100) longitudinally in a screw-like motion thereby preventing collapsing and/or vibrating and/or whipping of the distal section (104) of the microcatheter (100).

2. The embolization microcatheter of claim 1, wherein the off-setting portion (106) is curved.

3. The embolization microcatheter of any one of claims 1 and 2, wherein the longitudinal axis of the winding portion (108) is offset by between about 0.5 mm and about 5 mm relative to the longitudinal axis of the elongated main body (102).

4. The embolization microcatheter of any one of claims 1 to 3, wherein the acute angle, at which the longitudinal axis of the winding portion (108) is disposed relative to the longitudinal axis of the elongated main body (102), is between about 10° and about 60°.

5. The embolization microcatheter of any one of claims 1 to 4, wherein a length of the winding portion (108) measures between about 5 mm and about 20 mm.

6. The embolization microcatheter of any one of claims 1 to 5, wherein a ratio of the length of the winding portion (108) to a radius or mean radius, defined by the half a loop, is between about 2 and about 20.

7. The embolization microcatheter of any one of claims 1 to 6, wherein the three-dimensional spiral is right-handed; or wherein the three-dimensional spiral is left-handed.

8. The embolization microcatheter of any one of claims 1 to 7, wherein the three-dimensional spiral is a helix.

9. The embolization microcatheter of any one of claims 1 to 8, wherein the distal section (104) is pre-shaped.

10. The embolization microcatheter of any one of claims 1 to 9, wherein the main body (102) and the off-setting portion (106) together define a non-planar curve.

11. The embolization microcatheter of any one of claims 1 to 10, wherein the elongated main body (102) further comprises a filter section comprising a plurality of side openings formed in a wall of the elongated main body (102), preferably wherein the side openings are in the form of slits having a length between about 200 µm and about 800 µm and a width between about 25 µm and about 100 µm.

12. The embolization microcatheter of any one of claims 1 to 11, comprising a wall comprising an inner layer, an outer layer, and a skeleton intercalated between the inner layer and the outer layer, preferably wherein an outer layer section of the outer layer, on the distal section (104), is more flexible than an outer layer section of the outer layer, on the elongated main body (102).

13. The embolization microcatheter of any one of claims 1 to 12, wherein the distal section (104) is dimensioned such that, when inserted into a tortuous blood vessel, different portions of the distal section (104) contact opposite sides of the tortuous blood vessel at different longitudinal positions of the tortuous blood vessel.

14. The embolization microcatheter of any one of claims 1 to 13, configured for guide-wire free navigation.

15. A method for producing an embolization microcatheter (100) according to claim 1 with a distal section (104) that defines a non-planar curve, the method comprising:
providing a 3-dimensional mandrel comprising an off-setting portion (106) and a winding portion (108), wherein the winding portion (108) comprises about half a loop of a three-dimensional spiral, such that a longitudinal axis of the winding portion (108) is offset and/or disposed at an acute angle relative to a longitudinal axis of the elongated main body (102); and
wherein the off-setting portion (106) is disposed at an angle of between about 60° and about 120° degrees relative to the longitudinal axis of the elongated main body (102);
in a heat chamber forming the embolization microcatheter tube on the mandrel, thereby producing the embolization microcatheter (100) having the shape of the mandrel.

## Patentansprüche

1. Embolisationsmikrokatheter (100), der einen länglichen Hauptkörper (102) und einen distalen Abschnitt (104) umfasst, wobei der distale Abschnitt (104) einen versetzenden Teil (106) und einen Wicklungsteil (108) umfasst;
wobei der Wicklungsteil (108) etwa eine halbe Schleife einer dreidimensionalen Spirale umfasst, so dass eine Längsachse des Wicklungsteils (108) versetzt und/oder in einem spitzen Winkel relativ zu einer Längsachse des länglichen Hauptkörpers (102) angeordnet ist;
wobei der versetzende Teil (106) in einem Winkel zwischen etwa 60° und etwa 120° Grad relativ zu der Längsachse des länglichen Hauptkörpers (102) angeordnet ist, und
wobei der Wicklungsteil (108) dazu ausgestaltet ist, den Mikrokatheter (100) in Längsrichtung in einer schraubenartigen Bewegung zu manövrieren, wodurch Zusammenfallen und/oder Vibrieren und/oder Peitschenbewegung des distalen Abschnitts (104) des Mikrokatheters (100) verhindert wird.

2. Embolisationsmikrokatheter nach Anspruch 1, wobei der versetzende Teil (106) gekrümmt ist.

3. Embolisationsmikrokatheter nach einem der Ansprüche 1 und 2, wobei die Längsachse des Wicklungsteils (108) um zwischen etwa 0,5 mm und etwa 5 mm relativ zu der Längsachse des länglichen Hauptkörpers (102) versetzt ist.

4. Embolisationsmikrokatheter nach einem der Ansprüche 1 bis 3, wobei der spitze Winkel, in dem die Längsachse des Wicklungsteils (108) relativ zu der Längsachse des länglichen Hauptkörpers (102) angeordnet ist, zwischen etwa 10° und etwa 60° liegt.

5. Embolisationsmikrokatheter nach einem der Ansprüche 1 bis 4, wobei eine Länge des Wicklungsteils (108) zwischen etwa 5 mm und etwa 20 mm beträgt.

6. Embolisationsmikrokatheter nach einem der Ansprüche 1 bis 5, wobei ein Verhältnis der Länge des Wicklungsteils (108) zu einem Radius oder mittleren Radius, definiert durch die halbe Schleife, zwischen etwa 2 und etwa 20 liegt.

7. Embolisationsmikrokatheter nach einem der Ansprüche 1 bis 6, wobei die dreidimensionale Spirale rechtsdrehend ist; oder wobei die dreidimensionale Spirale linksdrehend ist.

8. Embolisationsmikrokatheter nach einem der Ansprüche 1 bis 7, wobei die dreidimensionale Spirale eine Helix ist.

9. Embolisationsmikrokatheter nach einem der Ansprüche 1 bis 8, wobei der distale Abschnitt (104) vorgeformt ist.

10. Embolisationsmikrokatheter nach einem der Ansprüche 1 bis 9, wobei der Hauptkörper (102) und der versetzende Teil (106) zusammen eine nicht-planare Kurve definieren.

11. Embolisationsmikrokatheter nach einem der Ansprüche 1 bis 10, wobei der längliche Hauptkörper (102) ferner einen Filterabschnitt umfasst, der mehrere Seitenöffnungen umfasst, die in einer Wand des länglichen Hauptkörpers (102) gebildet sind, wobei die Seitenöffnungen vorzugsweise in Form von Schlitzen mit einer Länge zwischen etwa 200 µm und etwa 800 µm und einer Breite zwischen etwa 25 µm und etwa 100 µm vorliegen.

12. Embolisationsmikrokatheter nach einem der Ansprüche 1 bis 11, der eine Wand umfasst, die eine Innenschicht, eine Außenschicht und ein Skelett umfasst, das zwischen der Innenschicht und der Außenschicht eingefügt worden ist, wobei vorzugsweise ein Außenschichtabschnitt der Außenschicht auf dem distalen Abschnitt (104) flexibler als ein Außenschichtabschnitt der Außenschicht auf dem länglichen Hauptkörper (102) ist.

13. Embolisationsmikrokatheter nach einem der Ansprüche 1 bis 12, wobei der distale Abschnitt (104) so dimensioniert ist, dass, wenn er in ein gewundenes Blutgefäß eingeführt wird, unterschiedliche Teile des distalen Abschnitts (104) gegenüberliegende Seiten des gewundenen Blutgefäßes an unterschiedlichen Längspositionen des gewundenen Blutgefäßes kontaktieren.

14. Embolisationsmikrokatheter nach einem der Ansprüche 1 bis 13, der für führungsdrahtfreie Navigation ausgestaltet ist.

15. Verfahren zum Produzieren eines Embolisationsmikrokatheters (100) nach Anspruch 1 mit einem distalen Abschnitt (104), der eine nicht-planare Kurve definiert, wobei das Verfahren Folgendes umfasst:
Bereitstellen eines 3-dimensionalen Dorns, der einen versetzenden Teil (106) und einen Wicklungsteil (108) umfasst, wobei der Wicklungsteil (108) etwa eine halbe Schleife einer dreidimensionalen Spirale umfasst, so dass eine Längsachse des Wicklungsteils (108) versetzt und/oder in einem spitzen Winkel relativ zu einer Längsachse des länglichen Hauptkörpers (102) angeordnet ist; und
wobei der versetzende Teil (106) in einem Winkel zwischen etwa 60° und etwa 120° Grad relativ zu der Längsachse des länglichen Hauptkörpers (102) angeordnet ist;
in einer Wärmekammer, worin das Embolisationsmikrokatheterrohr auf dem Dorn gebildet wird, wodurch der Embolisationsmikrokatheter (100) mit der Form des Dorns produziert wird.

## Revendications

1. Microcathéter d'embolisation (100) comprenant un corps principal allongé (102) et une section distale (104), la section distale (104) comprenant une partie de décalage (106) et une partie d'enroulement (108) ;
dans lequel la partie d'enroulement (108) comprend environ une demi-boucle d'une spirale tridimensionnelle, de telle sorte qu'un axe longitudinal de la partie d'enroulement (108) est décalé et/ou disposé à un angle aigu par rapport à un axe longitudinal du corps principal allongé (102) ;
dans lequel la partie de décalage (106) est disposée à un angle compris entre environ 60 et environ 120 degrés par rapport à l'axe longitudinal du corps principal allongé (102), et
dans lequel la partie d'enroulement (108) est conçue pour déplacer longitudinalement le microcathéter (100) selon un mouvement hélicoïdal, empêchant ainsi l'affaissement et/ou la vibration et/ou le fouettement de la section distale (104) du microcathéter (100).

2. Microcathéter d'embolisation selon la revendication 1, dans lequel la partie de décalage (106) est incurvée.

3. Microcathéter d'embolisation selon l'une quelconque des revendications 1 et 2, dans lequel l'axe longitudinal de la partie d'enroulement (108) est décalé d'environ 0,5 mm à environ 5 mm par rapport à l'axe longitudinal du corps principal allongé (102).

4. Microcathéter d'embolisation selon l'une quelconque des revendications 1 à 3, dans lequel l'angle aigu que fait l'axe longitudinal de la partie d'enroulement (108) par rapport à l'axe longitudinal du corps principal allongé (102) est compris entre environ 10° et environ 60°.

5. Microcathéter d'embolisation selon l'une quelconque des revendications 1 à 4, dans lequel la longueur de la partie d'enroulement (108) est comprise entre environ 5 mm et environ 20 mm.

6. Microcathéter d'embolisation selon l'une quelconque des revendications 1 à 5, dans lequel le rapport de la longueur de la partie d'enroulement (108) au rayon ou rayon moyen, défini par la demi-boucle, est compris entre environ 2 et environ 20.

7. Microcathéter d'embolisation selon l'une quelconque des revendications 1 à 6, dans lequel la spirale tridimensionnelle est dextrogyre ; ou dans lequel la spirale tridimensionnelle est lévogyre.

8. Microcathéter d'embolisation selon l'une quelconque des revendications 1 à 7, dans lequel la spirale tridimensionnelle est une hélice.

9. Microcathéter d'embolisation selon l'une quelconque des revendications 1 à 8, dans lequel la section distale (104) est préformée.

10. Microcathéter d'embolisation selon l'une quelconque des revendications 1 à 9, dans lequel le corps principal (102) et la partie de décalage (106) définissent ensemble une courbe non plane.

11. Microcathéter d'embolisation selon l'une quelconque des revendications 1 à 10, dans lequel le corps principal allongé (102) comprend en outre une section de filtre comprenant une pluralité d'ouvertures latérales formées dans une paroi du corps principal allongé (102), de préférence dans lequel les ouvertures latérales sont sous la forme de fentes ayant une longueur comprise entre environ 200 µm et environ 800 µm et une largeur comprise entre environ 25 µm et environ 100 µm.

12. Microcathéter d'embolisation selon l'une quelconque des revendications 1 à 11, comprenant une paroi comprenant une couche interne, une couche externe et un squelette intercalé entre la couche interne et la couche externe, de préférence dans lequel une section de couche externe de la couche externe, sur la section distale (104), est plus souple qu'une section de couche externe de la couche externe, sur le corps principal allongé (102).

13. Microcathéter d'embolisation selon l'une quelconque des revendications 1 à 12, dans lequel la section distale (104) est dimensionnée de telle sorte que, lorsqu'elle est insérée dans un vaisseau sanguin tortueux, différentes parties de la section distale (104) entrent en contact avec des côtés opposés du vaisseau sanguin tortueux à différentes positions longitudinales du vaisseau sanguin tortueux.

14. Microcathéter d'embolisation selon l'une quelconque des revendications 1 à 13, conçu pour une navigation sans fil-guide.

15. Procédé de production d'un microcathéter d'embolisation (100) selon la revendication 1, avec une section distale (104) qui définit une courbe non plane, le procédé comprenant :
la fourniture d'un mandrin tridimensionnel comprenant une partie de décalage (106) et une partie d'enroulement (108), la partie d'enroulement (108) comprenant environ une demi-boucle d'une spirale tridimensionnelle, de sorte qu'un axe longitudinal de la partie d'enroulement (108) est décalé et/ou disposé à un angle aigu par rapport à un axe longitudinal du corps principal allongé (102) ;
et
dans lequel la partie de décalage (106) est disposée à un angle compris entre environ 60 degrés et environ 120 degrés par rapport à l'axe longitudinal du corps principal allongé (102) ;
dans une chambre de chauffage formant le tube de microcathéter d'embolisation sur le mandrin, ce qui permet de produire le microcathéter d'embolisation (100) ayant la forme du mandrin.
